(19) Europäisches Patentamt
European Patent Office
Office européen des brevets

(11) Veröffentlichungsnummer: **0 098 802**
**B1**

(12) **EUROPÄISCHE PATENTSCHRIFT**

(45) Veröffentlichungstag der Patentschrift: **04.11.87**

(51) Int. Cl.⁴: **C 07 C 91/26,** A 61 K 7/08

(21) Anmeldenummer: **83810290.3**

(22) Anmeldetag: **29.06.83**

(54) **Verfahren zur Herstellung von quartären Ammoniumsalzen.**

(30) Priorität: **05.07.82 CH 4084/82**
**25.01.83 CH 396/83**

(43) Veröffentlichungstag der Anmeldung:
**18.01.84 Patentblatt 84/3**

(45) Bekanntmachung des Hinweises auf die Patenterteilung:
**04.11.87 Patentblatt 87/45**

(84) Benannte Vertragsstaaten:
**BE CH DE FR GB IT LI NL**

(56) Entgegenhaltungen:
**EP-A-0 075 065**

**RESEARCH DISCLOSURE, Band 227, März 1983, Artikel Nr. 22710, Havant, Hampshire, GB; "Cosmetic products containing quaternary ammonium salts"**

(73) Patentinhaber: **BASF Aktiengesellschaft, Carl-Bosch- Strasse 38, D-6700 Ludwigshafen (DE)**

(72) Erfinder: **Günter, Franz, Dr., Erlensträsschen 73, CH- 4125 Riehen (CH)**

**EP 0 098 802 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein Verfahren zur Herstellung von quartären Ammoniumsalzen.

Aus der EP-A-75 065, die nach Artikel 54 (3) EPÜ Stand der Technik ist, ist bekannt, quaternäre Ammoniumverbindungen durch Umsetzung von tertiären Aminen mit einer Epoxidverbindung, die wenigstens eine endständige Epoxygruppe aufweist z. B. einem Epoxyalkan oder Glycidether, herzustellen.

Aus der DE-A-646 339 ist weiterhin bekannt, quaternäre Ammoniumbasen durch Umsetzung von tertiären Aminen mit Äthylenoxid in Gegenwart von Wasser herzustellen. Gegebenenfalls muss dabei mit einem Überschuss an Äthylenoxid gearbeitet werden, damit eine weitgehende Umsetzung des Amins in die entsprechende Ammoniumbase erreicht wird. Ausgehend von primären oder sekundären Aminen erfolgt gemäss DE-B-2 052 321 die Reaktion mit Äthylenoxid zu quartären Ammoniumbasen in Gegenwart von Wasser, bei erhöhter Temperatur sowie durch Anwendung von Druck. Aus den erhaltenen Basen (Ammoniumhydroxide) lassen sich dann durch Umsetzung mit Säuren die entsprechenden quartären Ammoniumsalze erhalten.

In den beiden zuletzt genannten Verfahren zur Herstellung von quaternären Ammoniumverbindungen aus tertiären Aminen und Äthylenoxid fallen jedoch neben den gewünschten Ammoniumverbindungen grosse Mengen unerwünschter Nebenprodukte an. Beispielsweise können gemäss DE-A-646 339 die erhaltenen quartären Basen mit weiteren Mengen Äthylenoxid Kondensationsprodukte bilden. Dies ist besonders dann der Fall, wenn Äthylenoxid im Überschuss eingesetzt wird. Nach dem Verfahren gemäss DE-B-2 052 321 können auch schon die Ausgangsverbindungen Äthylenoxid addieren, was dann zur Bildung von tertiären Amin-Äthylenoxid-Addukten führt. Die Bildung solcher Nebenprodukte ist ein grosser Nachteil, da einerseits die Ausbeute an gewünschten Umsetzungsprodukten herabgesetzt wird und andererseits in der Regel umfangreiche Aufarbeitungsverfahren notwendig sind, um zu reinen Endprodukten zu gelangen.

Aufgabe der vorlielenden Erfindung ist daher, ein Verfahren bereitzustellen, gemäss dem quartäre Ammoniumsalze in hoher Reinheit und Ausbeute hergestellt werden können.

Die erfindungsgemässe Aufgabe wird dadurch gelöst, dass man tertiäre Amine in wässrigem Medium in Gegenwart von Säure mit Äthylenoxid quaterniert.

Gegenstand der vorliegenden Erfindung ist somit ein Verfahren zur Herstellung von Verbindungen der Formel

(1) 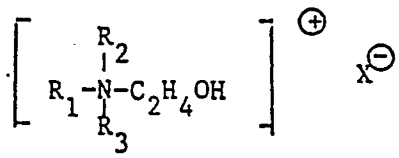 ,

worin $R_1$ Alkyl mit 8 bis 22 Kohlenstoffatomen ist, $R_2$ und $R_3$ je Alkyl mit 1 bis 4 Kohlenstoffatomen sind und $X^\ominus$ ein Anion ist, das dadurch gekennzeichnet ist dass man eine Verbindung der Formel

(2) 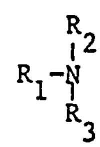 ,

worin $R_1$, $R_2$ und $R_3$ die angegebenen Bedeutungen haben, in wässrigem Medium, das mit einer Säure auf einen pH-Wert von 7,5 bis 9 eingestellt ist, mit Äthylenoxid bei 15 bis 90°C gegebenenfalls unter Druck quaterniert, durch Zugabe der gleichen Säure das Reaktionsgemisch auf einen pH-Wert im Bereich von 3 bis 7 einstellt und das erhaltene Ammoniumsalz gegebenenfalls isoliert.

Gegenstand der Erfindung sind ferner die nach diesen Verfahren erhaltenen Verbindungen, insbesondere die Phosphate, deren Verwendung in Haarkosmetika sowie wässrige oder wässrig-alkoholische Lösungen dieser Verbindungen.

In den Verbindungen der Formel (1) sind $R_2$ und $R_3$ z. B. Methyl, Äthyl, Propyl oder Butyl, wobei Methyl besonders bevorzugt ist. Für $R_1$ kommen beispielsweise Octyl, Nonyl, Decyl, Dodecyl, Hexadecyl, Octadecyl, Eicosyl und Docosyl in Frage. Geradkettige Alkylreste sind bevorzugt. Besonders geeignet sind Alkylreste mit 10 bis 16 Kohlenstoffatomen, wobei Hexadecyl besondere Bedeutung zukommt.

Als Säuren, die bei der Quaternierung der Amine der Formel (2) mit Äthylenoxid verwendet werden, können anorganische Säuren wie z. B. Chlor- und Bromwasserstoffsäure, Schwefel-, Phosphor- und Salpetersäure, sowie auch organische Säuren, z. B. niedermolekulare Mono-, Di- oder Tricarbonsäuren mit z. B. 1 bis 6, vorzugsweise 1 bis 4 Kohlenstoffatomen wie Ameisensäure, Essigsäure, Glykolsäure, Oxalsäure, Milchsäure und Citronensäure in Frage kommen. Demgemäss bedeutet $X^\ominus$ z. B. Chlorid, Bromid, Sulfat, Hydrogensulfat, Hydrogenphosphat, Dihydrogenphosphat, Nitrat, Formiat, Acetat, Glykolat, Oxalat, Lactat oder Citrat. Phosphorsäure eignet sich in besonderem Masse.

Vorzugsweise führt man die Quaternierung bei 60 bis 80° C durch.

Das erfindungsgemässe Verfahren lässt sich durch die Anwendung von Druck wesentlich beschleunigen. Geeignete Drucke liegen etwa im Bereich von 0,5 bis 1,5 bar Überdruck.

Die Anwesenheit einer bestimmten Menge Säure bei der Quaternierung der tertiären Amine mit Äthylenoxid ist die Grundvoraussetzung für eine praktisch quantitative, d.h. über 95 %ige Umsetzung des vorgelegten Amins. Verzichtet man dagegen auf den Zusatz von Säure so werden nur etwa 2/3 des vorgelegten Amins quaterniert. Dieser Umsatz lässt sich auch durch Anwendung eines 100 %igen Überschusses an Äthylenoxid nicht weiter steigern. Ferner nimmt bei Abwesenheit von Säure die Viskosität des Reaktionsgemisches während des Einleitens von Äthylenoxid sehr schnell zu. Bis zum Ende der Reaktion, d.h. nach etwa 2/3 des Umsatzes, bildet sich eine wachsartige Masse, die sich weder rühren noch umwälzen lässt. Findet jedoch die Quaternierung des Amins in Gegenwart von Säure statt, so verläuft die Reaktion fast quantitativ, und man erhält eine klare, leicht giessbare wässrige Lösung der quartären Ammoniumbase.

Geeignet sind solche Säuremengen, durch die die Reaktionslösung vor Einleiten des Äthylenoxids schwach alkalisch gestellt wird. Vorzugsweise liegt der pH-Wert in einen Bereich von 7,5 bis 9. Nach beendeter Reaktion wird dann mit derselben Säure die erhaltene Ammoniumbase in das entsprechende Ammoniumsalz überführt. Dabei verschiebt sich der pH-Wert der Lösung in den neutralen bis schwach sauren Bereich und nimmt vorzugsweise Werte von 3 bis 7 an. Wird die Gesamtmenge an Säure schon bei der Quaternierung eingesetzt, so können leich Hydrolyse- bzw. Esterverbindungen des Äthylenoxids entstehen, während sich quaternierte Ammoniumverbindungen nur in geringen Konzentrationen bilden.

In der Regel werden die erfindungsgemäss hergestellten Verbindungen nicht isoliert, sondern als wässrige Lösungen, wie sie nach Beendigung der Herstellung anfallen, eingesetzt.

Die erfindungsgemäss hergestellten Verbindungen eignen sich z. B. zur Verwendung in Haarkosmetika, insbesondere in Haarkonditioniermitteln.

Die Herstellung dieser Mittel aus den erfindungsgemäss hergestellten Verbindungen ist problemlos, da sie nicht nur mit Wasser sondern auch z. B. mit Wasser/Alkohol-Mischungen zu klaren, stabilen Lösungen führen, die die Haarbehandlung vereinfachen. Solche wässrigen Lösungen können 0,1 bis 1, vorzugsweise 0,2 bis 0,5 Gew.% an erfindungsgemäss hergestellten Verbindungen enthalten. Alkohol, vorzugsweise Äthanol oder i-Propanol, kann in Mengen von 10 bis 50, vorzugsweise 20 bis 30 Gew.% den wässrigen Lösungen zugesetzt werden. Insbesondere das Nachspülen der Haare wird durch solche die erfindungsgemäss hergestellten Verbindungen enthaltenden Lösungen vereinfacht. Die Verbindungen eignen sich ferner als Komponente in Zusammensetzungen, die z. B. für das Legen von Dauerwellen verwendet werden, und sie tragen auch zur Haltbarkeit der Frisur bei. Gegen Oxidationsmittel, wie z. B. Wasserstoffperoxid (vorzugsweise Lösungen mit einem pH-Wert von etwa 2,5) und Natriumbromat (vorzugsweise Lösungen mit einem pH-Wert von etwa 7,9) erweisen sich die Ammoniumsalze stabil, sodass ihre guten Konditioniereffekte auch z. B. beim oxidativen Färben der Haare zur Geltung kommen können.

Das folgende Beispiel dient zur Erläuterung der Erfindung, ohne sie darauf zu beschränken. Teile und Prozente beziehen sich auf das Gewicht, sofern nicht anders angegeben.

**Beispiel 1:**

0,5 Mol Dimethylhexadecylamin in 440 ml destilliertem Wasser werden vorgelegt. Nach Zugabe von 0,2 Mol 75 %iger Phosphorsäure erhält man eine klare Lösung, die auf 75 bis 80°C erhitzt wird. Dann leitet man während 60 Minuten 0,75 Mol Äthylenoxid ein. Anschliessend entfernt man die Heizung und gibt in das Reaktionsgemisch weitere 0,2 Mol 75 %ige Phosphorsäure und 23 ml destilliertes Wasser. Gegebenenfalls filtriert man anschliessend die Reaktionsmischung. Man erhält 665 bis 670 g einer klaren wässrigen Lösung, die etwa 30 % des Ammoniumsalzes der Formel

(101) 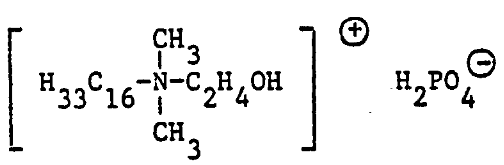

enthält. Dies entspricht einer Ausbeute von 96 bis 97 % an Verbindung der Formel (101).

Durch Anwendung von Überdruck erhöht sich die Geschwindigkeit der Äthylenoxidaufnahme. Bei etwa 0,5 bar Überdruck ist die Äthylenoxidaufnahme nach 20 Minuten, bei etwa 1,5 bar Überdruck schon nach 12 Minuten beendet. Die Ausbeuten an Verbindungen der Formel (101) sind ebenfalls fast quantitativ.

Die erhaltenen Lösungen können entweder direkt (als 30 %ige Lösung) oder in verdünnter Form in Haarkosmetika verwendet werden.

Die Verbindungen der Formel (101) lassen sich aus den wässrigen Lösungen durch z. B. Ausfrieren oder Zerstäubungstrocknen in fester Form erhalten.

**Beispiel 2:**

0,5 Mol Dimethylhexadecylamin in 440 ml destilliertem Wasser werden mit 0,25 Mol 25 %iger Schwefelsäure versetzt. Die so erhaltene Emulsion wird auf 75° C erhitzt. Ohne weitere Wärmezufuhr werden dann während 90 Minuten 0,7 Mol Äthylenoxid in die Emulsion geleitet. Durch gleichzeitiges Zutropfen von 25 %iger Schwefelsäure wird der pH-Wert zwischen 9 und 10 gehalten. Nach Einleiten des Äthylenoxids wird mit der Schwefelsäure ein pH-Wert von 5 eingestellt. Man erhält so 700 bis 710 g einer wässrigen, schwach trüben Lösung, die etwa 30 % des Ammoniumsalzes der Formel

(102) 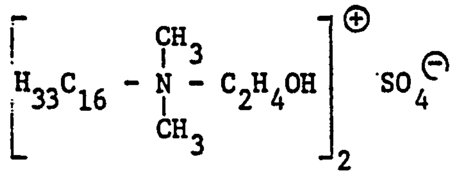

enthält.
Werden Dimethylhexadecylamin und Schwefelsäure im äquimolaren Verhältnis eingesetzt (z. B. 0,5 Mol Amin und 0,5 Mol Schwefelsäure) erhält man das Amoniumsalz der Formel

(102 a) 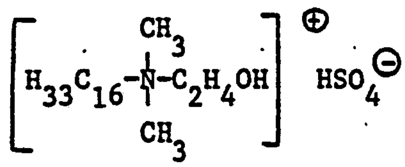

als etwa 30 %ige Lösung mit einem pH-Wert von 1,5-2.

**Beispiel 3:**

0,5 Mol Dimethylhexadecylamin in 400 ml destilliertem Wasser werden mit einer Lösung von 0,25 Mol d,1-Milchsäure in 22,5 ml destilliertem Wasser versetzt. Man erhält eine weisse Emulsion, die auf 75°C erwärmt wird. Dann werden während 2 bis 3 Stunden 0,75 Mol Äthylenoxid eingeleitet, wobei gleichzeitig eine Lösung von 0,25 Mol d,1-Milchsäure in 22,5 ml destilliertem Wasser in der Weise zugetropft wird, dass der pH-Wert der Emulsion immer 9 bis 10 beträgt. Da die Viskosität der Reaktionsmasse gegen Ende der Reaktion zunimmt, wird auf 90 bis 92° C erwärmt, um eine gute Rührbarkeit zu gewährleisten. Nach dem Einleiten des Äthylenoxids wird die erhaltene noch warme, dünnflüssige Lösung mit der Milchsäurelösung auf einen pH-Wert von 4,5 bis 5 eingestellt.
Man erhält 690 bis 700 g einer klaren wässrigen Lösung, die etwa 30 % des Ammoniumsalzes der Formel

(103) 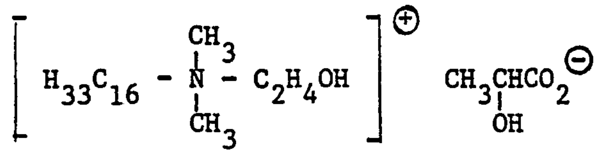

enthält und beim Abkühlen zu einem klaren Gel erstarrt.

**Beispiel 4:**

0,5 Mol Dimethylhexadecylamin in 355 ml destilliertem Wasser werden mit einer Lösung von 0,08 Mol Citronensäure (Monohydrat) in 32 ml destillierten Wasser versetzt. Man erhält eine weisse Emulsion, die auf 75°C erwärmt wird. Danach werden während 2 Stunden 0,75 Mol Äthylenoxid eingeleitet, wobei durch Zutropfen einer veiteren Lösung von 0,08 Mol Citronensäure (Monohydrat) in 32 ml destilliertem Wasser der pH-Wert der Reaktionsmasse zwischen 9 und 10 gehalten wird. Nach Einleiten des Äthylenoxids wird durch erneute Zugabe von Citronensäure (0,05 Mol gelöst in 15 ml destilliertem Wasser) der pH-Wert der Reaktionsmasse auf 5,5 gestellt.

Man erhält 645 bis 655 g einer klaren wässrigen Lösung, die etwa 30 % des Ammoniumsalzes der Formel

(104) 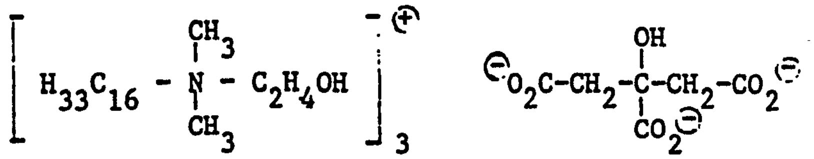

enthält.

Führt man die Reaktion mit insgesamt 0,5 Mol Citronensäure durch, so erhält man eine ebenfalls klare Lösung des Ammoniumsalzes der Formel

(105) 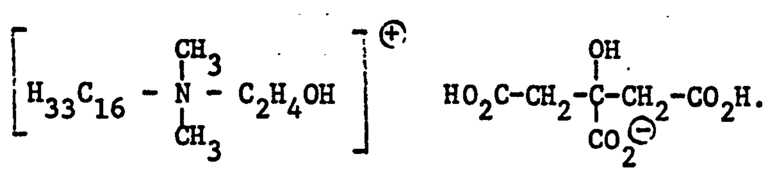

## Patentansprüche

1. Verfahren zur Herstellung von Verbindungen der Formel

(1) 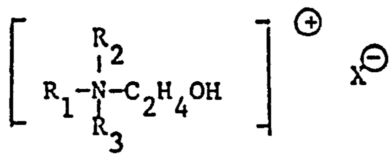 ,

worin $R_1$ Alkyl mit 8 bis 22 Kohlenstoffatomen ist, $R_2$ und $R_3$ je Alkyl mit 1 bis 4 Kohlenstoffatomen sind und $X^\ominus$ ein Anion ist, dadurch gekennzeichnet, dass man eine Verbindung der Formel

(2) 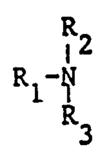 ,

worin $R_1$, $R_2$ und $R_3$ die angegebenen Bedeutungen haben, in wässrigem Medium das mit einer Säure auf einen pH-Wert von 7,5 bis 9 eingestellt ist, mit Äthylenoxid bei 15 bis 90°C gegebenenfalls unter Druck quaterniert, durch Zugabe der gleichen Säure das Reaktionsgemisch auf einen pH-Wert im Bereich von 3 bis 7 ein stellt und das erhaltene Ammoniumsalz gegebenenfalls isoliert.

2. Verfahren nach Anspruch 1, dadurch gekennzeichnet, dass $R_2$ und $R_3$ je Methyl sind.

3. Verfahren nach Anspruch 1, dadurch gekennzeichnet, dass die Säure Chlor- oder Bromwasserstoffsäure, Schwefel-, Phosphor- oder Salpetersäure oder eine niedermolekulare Mono-, Di- oder Tricarbonsäure ist.

4. Verfahren nach Anspruch 3, dadurch gekennzeichnet, dass die Säure Chlor- oder Bromwasserstoffsäure, Schwefel-, Phosphor- oder Salpetersäure oder eine niedermolekulare Mono- oder Dicarbonsäure ist.

5. Verfahren nach Anspruch 3, dadurch gekennzeichnet, dass die Säure Schwefel-, Phosphor-, Ameisen-, Essig-, Glykol-, Oxal-, Milch- oder Citronensäure ist.

6. Verfahren nach Anspruch 5, dadurch gekennzeichnet, dass die Säure Schwefel-, Phosphor-, Ameisen-, Essig-, Glykol- oder Oxalsäure ist.

7. Verfahren nach Anspruch 6, dadurch gekennzeichnet, dass die Säure Phosphorsäure ist.

8. Verfahren nach Anspruch 1, dadurch gekennzeichnet, dass man die Quaternierung bei 60 bis 80°C durchführt.

9. Verfahren nach Anspruch 1, dadurch gekennzeichnet, dass der Druck 0,5 bis 1,5 bar beträgt.

10. Die nach dem Verfahren nach Anspruch 1 erhaltenen Verbindungen.

11. Die Verbindung der Formel

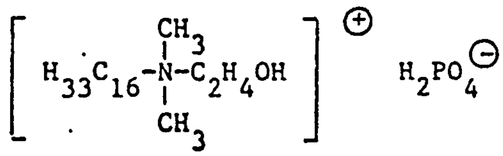

12. Verwendung der Verbindungen nach Anspruch 10 als Haarkonditioniermittel in Haarkosmetika.

13. Wässrige oder wässrig-alkoholische Lösungen der nach dem Verfahren gemäss Anspruch 1 erhaltenen Verbindungen.

## Claims

1. A process for preparing a compound of the formula

(1) 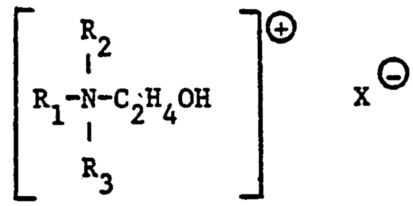

in which $R_1$ is alkyl having 8 to 22 carbon atoms, $R_2$ and $R_3$ are each alkyl having 1 to 4 carbon atoms and $X^{\ominus}$ is an anion, which comprises quaternising a compound of the formula

(2) 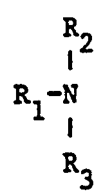 ,

in which $R_1$, $R_2$ and $R_3$ are as defined above, in an aqueous medium which has been adjusted to a pH-value of 7.5-9 by means of an acid, with ethylene oxide in the temperature range from 15° to 90° C and under pressure or at normal pressure, adjusting the

2. A process according to claim 1, wherein $R_2$ and $R_3$ are each methyl.

3. A process according to claim 1, wherein the acid is hydrochloric acid, hydrobromic acid, sulfuric acid, phosphoric acid, nitric acid or a low molecular weight mono-, di- or tri-carboxylic acid.

4. A process according to claim 3, wherein the acid is hydrochloric acid, hydrobromic acid, sulfuric acid, phosphoric acid, nitric acid or a low molecular weight mono- or dicarboxylic acid.

5. A process according to claim 3, wherein the acid is sulfuric phosphoric, formic, acetic, glycolic, oxalic, lactic or citric acid.

6. A process according to claim 5, wherein the acid is sulfuric, phosphoric, formic, acetic, glycolic or oxalic acid.

7. A process according to claim 6, wherein the acid is phosphoric acid.

8. A process according to claim 1 wherein the quaternisation is carried out in the temperature range from 60°

to 80°C.

9. A process according to claim 1, wherein the pressure is 0.5 to 1.5 bar.

10. A compound obtained by the process according to claim 1.

11. The compound of the formula

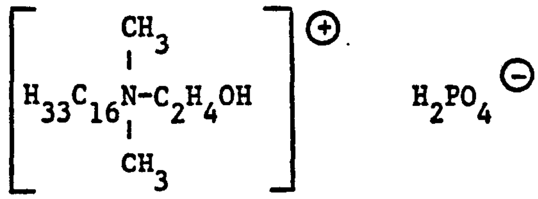

12. Use of a compound according to claim 10, as a hairconditioning agent in hair cosmetics.

13. An aqueous or aqueous-alcoholic solution of a compound obtained by the process according to claim 1.

**Revendications**

1. Procédé pour la préparation de composés de formule

(1) 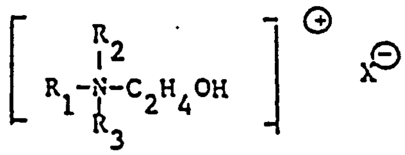

dans laquelle $R_1$ est un groupe alkyle ayant de 8 à 22 atomes de carbone, $R_2$ et $R_3$ sont chacun un groupe alkyle ayant de 1 à 4 atomes de carbone, et X est un anion, caractérisé par le fait que l'on effectue la quaternisation d'un composé de formule

(2) 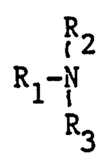

dans laquelle $R_1$, $R_2$ et $R_3$ ont les significations données plus haut, avec de l'oxyde d'éthylène, à 15-90°C, éventuellement sous pression, dans un milieu aqueux qui est ajusté à un pH de 7,5 à 9 au moyen d'un acide, on ajuste le mélange réactionnel à un pH situé dans l'intervalle de 3 à 7, par addition du même acide, et éventuellement on isole le sel d'ammonium obtenu.

2. Procédé selon la revendication 1, caractérisé par le fait que $R_2$ et $R_3$ sont chacun le groupe méthyle.

3. Procédé selon la revendication 1, caractérisé par le fait que l'acide est l'acide chlorhydrique ou bromhydrique, l'acide sulfurique, phosphorique ou nitrique, ou un acide mono-, di- ou tricarboxylique inférieur.

4. Procédé selon la revendication 3, caractérisé par le fait que l'acide est l'acide chlorhydrique ou bromhydrique, l'acide sulfurique, phosphorique ou nitrique, ou un acide mono- ou dicarboxylique inférieur.

5. Procédé selon la revendication 3, caractérisé par le fait que l'acide est l'acide sulfurique, phosphorique, formique, acétique, glycolique, oxalique, lactique ou citrique.

6. Procédé selon la revendication 5, caractérisé par le fait que l'acide est l'acide sulfurique, phosphorique, formique, acétique, glycolique ou oxalique.

7. Procédé selon la revendication 6, caractérisé par le fait que l'acide est l'acide phosphorique.

8. Procédé selon la revendication 1, caractérisé par le fait que l'on effectue la quaternisation à 60-80°C.

9. Procédé selon la revendication 1, caractérisé par le fait que la pression est de 0,5 à 1,5 bar.

10. Composés obtenus par le procédé selon la revendication 1.

11. Composé de formule

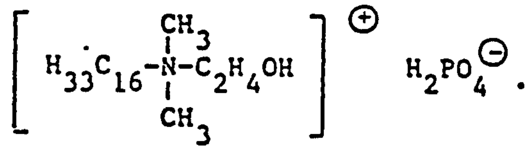

12. Utilisation des composés selon la revendication 10, en tant qu'agents de conditionnement du cheveu, dans des cosmétiques capillaires.

13. Solutions aqueuses ou aqueuses-alcooliques des composés obtenus par le procédé selon la revendication 1.